# EUROPÄISCHE PATENTSCHRIFT

(11) **EP 4 008 384 B1**
(45) Veröffentlichungstag und Bekanntmachung des Hinweises auf die Patenterteilung: **11.01.2023**
(21) Anmeldenummer: 21211191.8
(22) Anmeldetag: 30.11.2021
(51) Int. Cl.: A61M 16/10, B01D 46/00

(54) **EINWEGFILTER MIT AUFDRUCK**
SINGLE-USE FILTER WITH PRINTING
FILTRE JETABLE IMPRIMÉ

(30) Priorität: 04.12.2020 DE 102020132320
(43) Veröffentlichungstag der Anmeldung: 08.06.2022
(73) Patentinhaber: Drägerwerk AG & Co. KGaA, 23558 Lübeck (DE)
(72) Erfinder: Ise, Edgar, 23558 Lübeck (DE); Hampe, Markus, 23558 Lübeck (DE)

(56) Entgegenhaltungen:
- EP-A2- 0 754 470
- WO-A1-93/16749
- DE-A1-102019 107 316
- DE-U1-202010 014 742
- US-A- 6 051 042
- US-A1- 2012 198 804
- US-A1- 2017 002 205
- US-A1- 2019 107 302

## Beschreibung

Die Erfindung betrifft einen Einwegfilter zur Verwendung in einem Atemkreis eines Beatmungsgerätes. Weiterhin betrifft die Erfindung ein Verfahren zum Bedrucken eines Einwegfilters.

Grundsätzlich sind aus der Anwendung im Bereich der Intensivmedizin und der Anästhesie Einwegfilter bekannt, die zwischen Beatmungsschlauch und Patient oder zwischen Beatmungsschlauch und Beatmungsgerät in einem Atemkreis angeordnet sind. Diese Einwegfilter reinigen das Atemgas, das zum Patienten geht oder vom Patienten kommt, in verschiedenen Qualitätsgraden von Partikeln und Mikroben und/oder unterstützen ein Rückbefeuchten dieses Atemgases. In diesem Zusammenhang sind beispielsweise mechanische Filter, elektrostatische Filter und/oder HME (Heat Moisture Exchanger) Filter bekannt. Dokumente EP0754470 A2 und WO93/16749 A1 offenbaren beispielhafte Filter mit den Merkmalen wie in dem Oberbegriff des Anspruchs 1.

Insbesondere bei langzeitbeatmeten Patienten ist es wichtig zu wissen, wie lange ein solcher Filter schon am Patienten im Einsatz ist und demzufolge gegen einen neuen Filter getauscht werden muss, um eine ausreichende Filterwirkung zu gewährleisten, den pneumatischen Filterwiderstand gering zu halten und eine übermäßige Verkeimung des Filters zu vermeiden.

Bekannt ist hierbei der Einsatz von Aufklebern, die auf einen Einwegfilter geklebt werden können, um darauf das Datum und die Uhrzeit zu vermerken, an dem der Einwegfilter eingesetzt wurde. Hierdurch kann stets zurückverfolgt werden, wie lange der Filter bisher im Einsatz ist und ob ein Wechsel des Filters aufgrund seiner Einsatzzeit notwendig ist.

Aufgabe der vorliegenden Erfindung ist es, einen verbesserten Einwegfilter, insbesondere einen Einwegfilter mit einer verbesserten Kennzeichnungsmöglichkeit, bereitzustellen.

Erfindungsgemäß wird zur Lösung dieser Aufgabe ein Einwegfilter zur Verwendung in einem Atemkreis eines Beatmungsgerätes vorgeschlagen, mit einem Filtergehäuse und einem Aufdruck auf dem Filtergehäuse.

Das Filtergehäuse umgibt dabei ein Filtermaterial des Einwegfilters, wobei das Filtergehäuse eine erste Filteröffnung und eine zweite Filteröffnung zum jeweiligen Anschluss eines gasführenden Bauteils, insbesondere eines jeweiligen Schlauchs, für ein durch den Einwegfilter zu führendes Gas aufweist. Dabei ist das Filtermaterial zwischen der ersten Filteröffnung und der zweiten Filteröffnung angeordnet.

Der Aufdruck auf dem Filtergehäuse ist über ein Druckverfahren, insbesondere ein Tampondruckverfahren, auf dem Filtergehäuse angeordnet und bildet dabei eine strukturierte Fläche, wobei die strukturierte Fläche beschreibbar ist, und wobei die strukturierte Fläche mindestens sieben Ankreuzbereiche umfasst.

Im Rahmen der Erfindung wurde erkannt, dass der manuelle Aufwand zum Kennzeichen des Einwegfilters möglichst gering sein sollte, um das Pflegepersonal bei dieser regelmäßig notwendigen Kennzeichnung zu entlasten. Um dieses Problem zu lösen wird erfindungsgemäß vorgeschlagen, einen Aufdruck auf dem Filtergehäuse vorzusehen, der beschreibbar ist. Zudem ermöglichen Ankreuzbereiche eine komfortable Kennzeichnung, an welchem Tag der Einwegfilter eingesetzt wurde, ohne dass ein Datum aufgeschrieben zu werden braucht.

Der erfindungsgemäße Einwegfilter ermöglicht dadurch ein unmittelbares Beschreiben der strukturierten Fläche des Einwegfilters, sodass Aufkleber nicht von dem Pflegepersonal mitgeführt zu werden brauchen und auch nicht manuell auf den Einwegfilter aufgebracht werden müssen.

Das Vorsehen von sieben Ankreuzbereichen ermöglicht zudem vorteilhaft ein Kennzeichnen desjenigen Tages, an dem der Einwegfilter eingesetzt wurde, ohne dass ein Datum ausgeschrieben werden muss. Hierdurch wird die Kennzeichnung des Einwegfilters außerdem besonders zuverlässig, da Fehler beim Aufschreiben des Datums nicht auftreten können. So kennt eine Person typischerweise den aktuell vorliegenden Tag, wohingegen das Rekapitulieren des aktuellen Datums einen gewissen kognitiven Aufwand bedeutet. Zudem kann die Kennzeichnung des Datums unabhängig von sprachlichen Differenzen oder unterschiedlichen nationalen Besonderheiten bei der Schreibweise des Datums standardisiert erfolgen.

Die sieben Ankreuzbereiche indizieren erfindungsgemäß sieben Wochentage, sodass durch das Ankreuzen eines Ankreuzbereiches aus diesen sieben Ankreuzbereichen der aktuell vorliegende Tag angezeigt werden kann. Das Vorsehen eines Ankreuzbereiches zum Anzeigen eines Einsatztages des entsprechenden Einwegfilters ermöglicht zudem ein besonders schnelles Erfassen dieses Einsatztages, ohne dass das Erkennen einer Mehrzahl von Zahlen, die für das Ausschreiben eines Datums verwendet werden müssen, notwendig ist. Vorzugsweise ist jeder der sieben Ankreuzbereiche beschriftet, um sicherzustellen, dass alle den Filter einsetzenden Personen den gleichen Wochentag mit dem entsprechenden Ankreuzbereich assoziieren.

Eine strukturierte Fläche ist im Sinne der Erfindung dadurch strukturiert, dass die Fläche eine visuell erfassbare Struktur bildet. Die Fläche ist bereits dadurch strukturiert, dass sie die sieben Ankreuzbereiche aufweist.

Das Tampondruckverfahren ist dem Fachmann allgemein bekannt und wird daher nicht im Folgenden detailliert erläutert. Für den erfindungsgemäßen Einwegfilter ist die Verwendung des Tampondruckverfahrens dahingehend vorteilhaft, dass ein bekanntes Verfahren genutzt wird, welches vor allem für gekrümmte Oberflächen ein zuverlässiges Drucken ermöglicht. Mithin ergibt sich dadurch ein zuverlässiges Aufdrucken und eine kostengünstige Herstellung des Einwegfilters.

In alternativen erfindungsgemäßen Ausgestaltungen wird ein Siebdruck- oder ein Digitaldruckverfahren oder ein Heißprägeverfahren für den Aufdruck verwendet.

Die Beschreibbarkeit der strukturierten Fläche des Aufdrucks bedeutet, dass der Aufdruck beschrieben, vorzugsweise irreversibel beschrieben werden kann. Derartige beschreibbare Aufdrucke sind grundsätzlich bekannt, so dass im Folgenden nicht auf bekannte Charakteristiken des Aufdrucks eingegangen wird, die eine solche Beschreibbarkeit ermöglichen.

Unter einem Beatmungsgerät ist im Sinne der Erfindung ein Gerät zu verstehen, welches die Beatmung eines zu beatmenden Patienten unterstützt. Dies kann neben einem klassischen Beatmungsgerät beispielsweise auch ein Anästhesiegerät oder dergleichen sein.

Nachfolgend werden bevorzugte Ausführungsformen des erfindungsgemäßen Einwegfilters beschrieben.

Vorzugsweise ist der erfindungsgemäße Einwegfilter ein mechanischer Filter, ein elekrostatischer Filter, ein HME-Filter oder eine Kombination aus zwei oder drei der vorgenannten Filter. Die Verwendung des erfindungsgemäßen Aufdrucks für derartige Filter, insbesondere für ein HME-Filter ist besonders vorteilhaft, weil derartige Einwegfilter lediglich für eine geringe Nutzungsdauer ausgelegt sind. So sollte ein HME-Filter beispielsweise bereits nach 24 Stunden gewechselt werden, um eine ausreichende Filterwirkung zu gewährleisten und eine übermäßige Verkeimung zu vermeiden.

In einer bevorzugten Ausführungsform weist die strukturierte Fläche eine derartige Rauigkeit auf, dass sie mit einem Kugelschreiber beschreibbar ist. Die Beschreibbarkeit mit einem Kugelschreiber ermöglicht vorteilhaft die Verwendung eines beliebigen mitgeführten Kugelschreibers. Dies ist dadurch vorteilhaft, dass Krankenhauspersonal typischerweise einen Kugelschreiber mit sich führt. Hierdurch wird die umständliche Verwendung eines speziell für den Aufdruck vorgesehenen Schreibgerätes vermieden.

In einer weiteren vorteilhaften Ausführungsform umfasst die strukturierte Fläche zusätzlich zu den sieben Ankreuzbereichen einen runden Uhrenbereich, über den eine Uhrzeit auf der strukturierten Fläche angestrichen werden kann. In dieser Ausführungsform kann nicht nur das Datum des Einsatzes des entsprechenden Filters über einen der sieben Ankreuzbereiche indiziert werden, sondern es kann über ein Anstreichen, ein Kreisen und/oder Ankreuzen eines Teils des Uhrenbereiches eine Uhrzeit des Einsatzes dieses Filters angezeigt werden. Dies ist insbesondere vorteilhaft für einen Filter, der bereits nach wenigen Stunden gewechselt werden muss. So kann in dieser Ausführungsform besonders schnell das Datum und die Uhrzeit des Einsatzes des Filters gekennzeichnet werden und es können diese Informationen zudem besonders schnell optisch erfasst werden, um einzuschätzen, ob ein Wechsel des entsprechenden Einwegfilters notwendig ist.

In einer weiteren Ausführungsform ist die strukturierte Fläche eine zusammenhängende Fläche. In dieser Ausführungsform kann die strukturierte Fläche besonders einfach optisch erfasst werden, da nicht unterschiedliche Bereiche einer nicht zusammenhängenden Fläche ausgewertet werden müssen. Zudem erlaubt der Aufdruck gemäß dieser Ausführungsform eine besonders günstige Herstellung, da nicht mehrere Aufdrucke separat voneinander aufgebracht werden müssen.

In einer bevorzugten Variante der vorhergehenden Ausführungsform ist die strukturierte Fläche eine die erste oder die zweite Filteröffnung zumindest teilweise zirkular umgebende Fläche. Für eine derart geformte Fläche ist das Vorsehen eines Tampondruckverfahrens besonders vorteilhaft, da ein manuelles Aufkleben eines zirkular umlaufenden Aufklebers einen gewissen Aufwand für die Pflegekraft bedeuten würde. Zudem ist das Vorsehen des Tampondruckverfahrens besonders vorteilhaften, da ein zirkular umlaufender Aufdruck für ein Gehäuse typischerweise über eine gewölbte Fläche verläuft und daher ein zuverlässiges Drucken über eine derartige Fläche besonders einfach über das Tampondruckverfahren möglich ist. Durch die zirkular verlaufende strukturierte Fläche kann besonders schnell der für die Kennzeichnung des Einsatzdatums des Filters relevante Bereich der strukturierten Fläche optisch erfasst werden. So lenkt ein großer Aufdruck die Aufmerksamkeit des Nutzers schneller auf diesen Aufdruck, als wenn beispielsweise auf einer Seitenfläche des Filters ein Aufdruck erst gesucht werden muss.

Besonders bevorzugt umfasst die strukturierte Fläche eine Produktkennzeichnung für den Einwegfilter. In dieser Ausführungsform kann zusammen mit dem Vorsehen des Aufdrucks für die Kennzeichnung des Anwendungsdatums auch die Kennzeichnung auf dem Einwegfilter vorgesehen werden. So kann ein zusätzlicher Herstellungsschritt für das Aufbringen der Produktkennzeichnung eingespart werden.

In einer besonders bevorzugten Ausführungsform ist das Filtermaterial durch eine nicht lösbare Struktur des Filtergehäuses dauerhaft in dem Filtergehäuse verbaut. Das Filtergehäuse gemäß dieser Ausführungsform kann besonders vorteilhaft über den erfindungsgemäßen Aufdruck gekennzeichnet werden. So ist ein Filter mit einem dauerhaft verbauten Filtermaterial vorzugsweise ein Einwegprodukt, bei dem keine erneute Kennzeichnung über die strukturierte Fläche notwendig ist, da die entsprechend gekennzeichnete Fläche nach einmaliger Nutzung entsorgt wird. Alternativ kann der Einwegfilter ein Filter sein, bei dem das Filtermaterial ein Einwegmaterial ist, welches jedoch für eine zukünftige Verwendung ausgewechselt werden kann.

In einer besonders bevorzugten Ausführungsform ist der Einwegfilter in einer separaten irreversibel zu öffnenden Verpackung angeordnet, bevor er erstmalig verwendet wird. Die irreversibel zu öffnende Verpackung ist vorzugsweise ein verschweißter Folienbeutel. Alternativ oder ergänzend weist der Einwegfilter einen jeweiligen Verschluss zum Verschließen der ersten und/oder zweiten Filteröffnung vor einer erstmaligen Nutzung des Einwegfilters auf. Das Vorsehen eines jeweiligen Verschlusses stellt sicher, dass das Filtermaterial erst ab dem auf der strukturierten Fläche gekennzeichneten Tag verwendet wurde und nicht bereits vorher, beispielsweise durch Umgebungsluft, einem Gas ausgesetzt war. In dieser Ausführungsform kann über eine entsprechende Kennzeichnung der strukturierten Fläche, insbesondere eines Ankreuzbereiches, angezeigt werden, ab wann das Filtermaterial erstmalig einem Gas ausgesetzt gewesen ist.

In einer vorteilhaften Ausführungsform indiziert eine Farbe des Aufdrucks einen Filter-Typ des Einwegfilters aus einer vorbestimmten Gruppe von Filter-Typen. In dieser Ausführungsform hat der Aufdruck neben der Kennzeichnung, wann der Filter erstmalig eingesetzt wurde, auch die Funktion aufgrund seiner Farbe ein schnelles Erfassen des vorliegenden Filter-Typs zu ermöglichen. Besonders relevante Filter-Typen sind beispielsweise HME-Filter, elektrostatische Filter, mechanische Filter und/oder eine Kombination aus mechanischem Filter und HME-Filter.

In einer besonders vorteilhaften Ausführungsform umfasst die strukturierte Fläche ein ikonographisches Symbol, das eine Beschreibbarkeit dieser Fläche für einen Anwender des Einwegfilters anzeigt. Ein derartiges ikonographisches Symbol ist beispielsweise ein stilisierter Stift. Das Vorsehen eines derartigen ikonographischen Symbols ermöglicht ein schnelles optisches Erfassen der möglichen Beschreibbarkeit des Aufdrucks durch den Anwender des Einwegfilters. Hierdurch kann schnell erfasst werden, dass eine Fläche tatsächlich beschreibbar ist, insbesondere wo eine Fläche beschrieben werden sollte, sodass nicht hilfsweise trotz der Beschreibbarkeit des Filtergehäuses auf einen Aufkleber gemäß dem bekannten Stand der Technik zurückgegriffen wird.

Gemäß einem weiteren Aspekt der Erfindung wird zur Lösung der oben genannten Aufgabe ein Verfahren zum Bedrucken eines Einwegfilters vorgeschlagen, welches die folgenden Schritte aufweist:
- Bereitstellen eines Filtergehäuses; und
- Bedrucken des Filtergehäuses über ein Druckverfahren, insbesondere ein Tampondruckverfahren, derart, dass ein entsprechender Aufdruck eine strukturierte Fläche bildet, wobei die strukturierte Fläche beschreibbar ist, und wobei die strukturierte Fläche mindestens sieben Ankreuzbereiche umfasst.

Der erfindungsgemäße Einwegfilter wird durch das erfindungsgemäße Verfahren hergestellt, sodass dieses Verfahren sämtliche Vorteile des Einwegfilters aufweist. Insbesondere ermöglicht das erfindungsgemäße Verfahren die besonders einfache Herstellung eines Aufdrucks, durch welchen ein Einsatzdatum eines Einwegfilters schnell und einfach vermerkt werden kann, ohne einen Aufkleber aufkleben oder ein Datum ausschreiben zu müssen.

Weiterhin ermöglicht das Verfahren die Erstellung eines Aufdrucks, welcher besonders einfach und schnell optisch erfasst werden kann, um zu entscheiden, ob der entsprechende Einwegfilter ausgewechselt werden muss.

Die Erfindung soll nun anhand von in den Figuren schematisch dargestellten, vorteilhaften Ausführungsbeispielen näher erläutert werden. Von diesen zeigen im Einzelnen:
- Fig. 1: eine perspektivische Darstellung eines ersten Ausführungsbeispiels eines erfindungsgemäßen Einwegfilters;
- Fig. 2: eine Darstellung eines Aufdrucks des erfindungsgemäßen Einwegfilters gemäß dem ersten Ausführungsbeispiel;
- Fig. 3, 4: eine jeweilige Darstellung eines Aufdrucks eines erfindungsgemäßen Einwegfilters gemäß einem zweiten Ausführungsbeispiel (Fig. 3) und einem dritten Ausführungsbeispiel (Fig. 4); und
- Fig. 5: ein Flussdiagramm eines Ausführungsbeispiels eines erfindungsgemäßen Verfahrens.

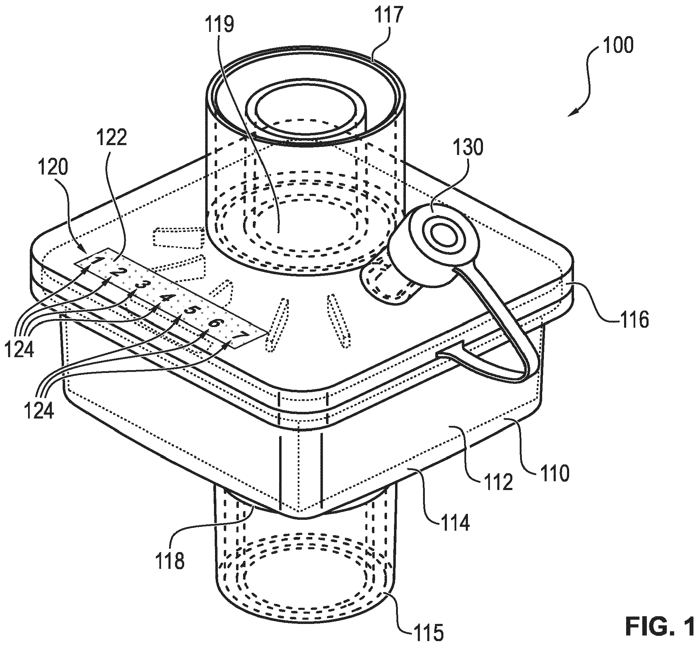

Fig.1 zeigt eine perspektivische Darstellung eines ersten Ausführungsbeispiels eines erfindungsgemäßen Einwegfilters 100.

Der Einwegfilter 100 ist zur Verwendung in einem Atemkreis eines Beatmungsgerätes ausgebildet. Hierfür weist der Einwegfilter 100 ein gebräuchliches Filtergehäuse 110 auf, welches ein Filtermaterial 112 des Einwegfilters 100 umgibt. In dem dargestellten Ausführungsbeispiel besteht das Filtergehäuse 110 aus einer ersten Gehäusekomponente 114 mit einem ersten Anschluss 115 für einen Schlauch oder ein Y-Stück und einer zweiten Gehäusekomponente 116 mit einem zweiten Anschluss 117 für ein gasführendes Bauteil, vorliegend einen Schlauch oder einen geräteseitigen Anschluss. Die beiden Gehäusekomponenten 114, 116 sind über eine Verschweißung, insbesondere über ein Ultraschall-Schweißen, formschlüssig miteinander verbunden. In einem alternativen Ausführungsbeispiel sind Gehäusekomponenten des Filtergehäuses über eine Verklebung, einen abgedichteten Einrastmechanismus oder dergleichen dauerhaft, insbesondere nicht-lösbar, miteinander verbunden. Zum Vorsehen der beiden Anschlüsse 115, 117 ist jeweils eine Filteröffnung 118, 119 in den entsprechenden Gehäusekomponenten 114, 116 vorgesehen. Dabei sind die beiden Filteröffnungen 118, 119 derart angeordnet, dass das Filtermaterial 112 zwischen der ersten Filteröffnung 118 und der zweiten Filteröffnung 119 liegt. Die beiden Anschlüsse 115, 117 sind in dem dargestellten Ausführungsbeispiel mit mehreren koaxialen Schnittstellen für Schläuche, ein Y-Stück oder einen geräteseitigen Anschluss ausgebildet. Das Filtergehäuse 110 ist vorzugsweise aus Polypropylen gebildet.

Erfindungsgemäß ist ein Aufdruck 120 auf dem Filtergehäuse 110 vorgesehen, der über ein Tampondruckverfahren auf dem Filtergehäuse 110 angeordnet wurde. Dieser Aufdruck 120 bildet eine strukturierte Fläche 122, also eine Fläche, die sich visuell in eine gewisse Struktur untergliedert, nämlich vorliegend unter anderem in sieben Ankreuzbereiche 124. Die strukturierte Fläche 122 ist dabei beschreibbar. Das Vorsehen eines Tampondrucks mit einer entsprechenden Druckcharakteristik um eine beschreibbar Fläche zu ermöglichen, ist bereits bekannt und wird daher nicht im Folgenden detailliert erläutert.

In dem dargestellten Ausführungsbeispiel werden die sieben Ankreuzbereiche 124 durch sieben Zahlen von 1 bis 7 gebildet, die die sieben Wochentage repräsentieren. In einem alternativen oder ergänzenden Ausführungsbeispiel werden die Ankreuzbereiche gebildet oder ergänzt durch jeweils mindestens einen Buchstaben, der den zu diesem Ankreuzbereich korrespondierenden Tag indiziert.

Über die dargestellten Ankreuzbereiche kann schnell und intuitiv vermerkt werden, wann der entsprechende Einwegfilter 100 eingesetzt wurde.

Die strukturierte Fläche 122 ist dabei mit einem Kugelschreiber beschreibbar. Sie weist also eine derartige Rauigkeit auf, dass eine Beschreibbarkeit mittels Kugelschreiber möglich ist.

Weiterhin ist dargestellt, dass ein Drainageverschluss 130 zum Verschließen einer korrespondierenden Drainageöffnung vorgesehen ist. Vor einer erstmaligen Nutzung des Einwegfilters 100 ist dieser Drainageverschluss 130 vorzugsweise verschlossen. Die zusätzliche Öffnung bietet dem Anwender über einen entsprechenden Anschluss die Möglichkeit, Kondenswasser aus dem Filter abzusaugen, um eine Einsatzdauer des Filters zu erhöhen, da dieser somit nicht nach dem Ansammeln einer kritischen Menge von Wasser gewechselt werden braucht. In einem nicht dargestellten Ausführungsbeispiel ist zumindest für den ersten Anschluss ein Verschluss zum Verschließen der entsprechenden Filteröffnung vorgesehen. Vor einer erstmaligen Nutzung des Einwegfilters ist dieser Verschluss vorzugsweise verschlossen

In einem nicht dargestellten Ausführungsbeispiel ist der Verschluss vor der erstmaligen Nutzung über ein verklebtes Siegel auf dem Anschluss gesichert. Das Siegel sichert dabei, dass der Verschluss nicht nach der Herstellung versehentlich abgegangen ist, sodass der Einwegfilter in seiner Funktionsfähigkeit möglicherweise bereits eingeschränkt wäre.

Vorzugsweise zeigt die Farbe der strukturierten Fläche 122 einen Filter-Typ des Einwegfilters 100 aus einer vorbestimmten Gruppe von Filter-Typen. In dem dargestellten Ausführungsbeispiel handelt es sich bei dem Einwegfilter 100 um einen mechanischen Filter. Alternativ oder ergänzend kann der Filter ein elektrostatischer Filter oder ein HME-Filter sein. Derartige Filter-Typen haben vorzugsweise einen andersfarbigen Aufdruck 120 als der dargestellte mechanische Filter.

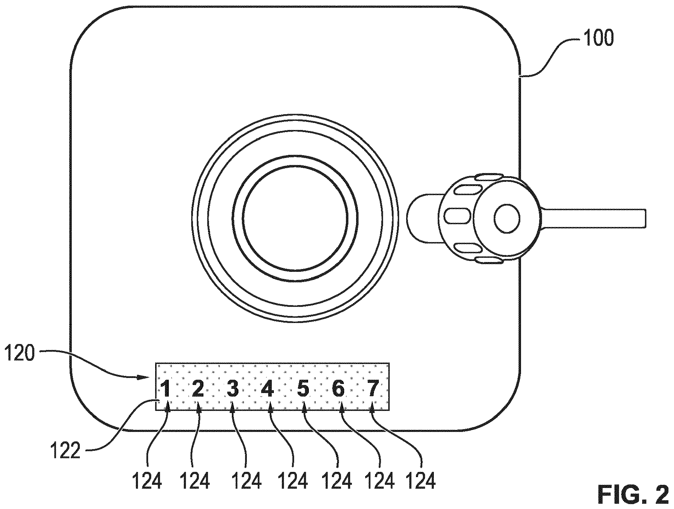

Fig. 2 zeigt eine Darstellung eines Aufdrucks 120 des erfindungsgemäßen Einwegfilters 100 gemäß dem ersten Ausführungsbeispiel.

Diese Darstellung veranschaulicht, dass die Ankreuzbereiche 124 schnell und einfach erkannt und angekreuzt werden können, nachdem der Einwegfilter 100 bei einem Patienten eingesetzt wurde. Weiterhin veranschaulicht diese Darstellung, dass ein Ankreuzbereich, der angekreuzt, abgehackt, eingekreist oder in ähnlicher Weise markiert wurde, schnell und eindeutig von dem entsprechenden Pflegepersonal erfasst werden kann, um zu entscheiden, ob der Einwegfilter ausgetauscht werden muss oder nicht.

In dem dargestellten Ausführungsbeispiel kann die Zahl 1 beispielsweise dem Montag entsprechen, und die Zahl 7 dem Sonntag. So kann über die markierte Zahl schnell erfasst werden, an welchem Tag der Einwegfilter 100 eingesetzt wurde.

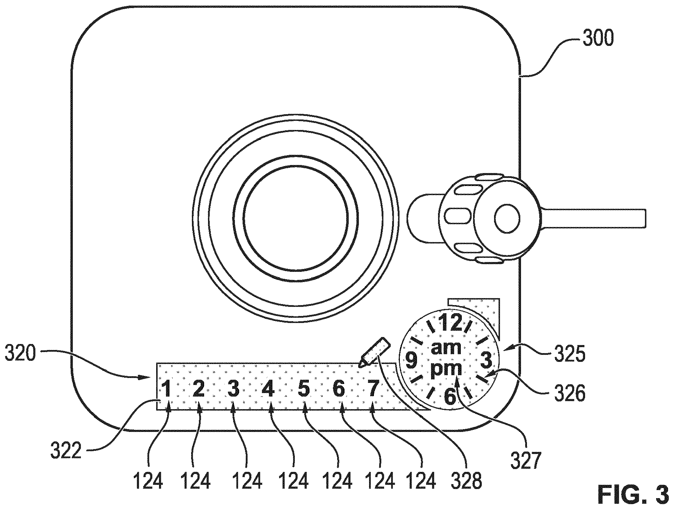

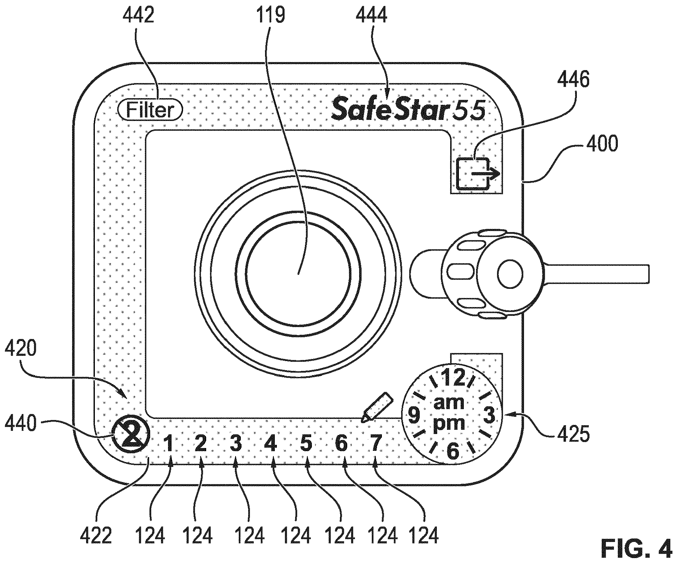

Die Figuren 3 und 4 zeigen eine jeweilige Darstellung eines Aufdrucks 320, 420 eines erfindungsgemäßen Einwegfilters 300, 400 gemäß einem zweiten Ausführungsbeispiel und einem dritten Ausführungsbeispiel.

Der in Fig. 3 dargestellte Einwegfilter 300 unterscheidet sich von dem Einwegfilter 100 ausschließlich durch seinen Aufdruck 320.

Der Aufdruck 320 umfasst zusätzlich zu den sieben Ankreuzbereichen 124 einen runden Uhrenbereich 325, über den eine Uhrzeit vermerkt werden kann, zu der der Einwegfilter 300 eingesetzt wurde. Hierfür verfügt der Uhrenbereich 325 über eine Stundenangabe 326 von 1:00 Uhr bis 12:00 Uhr und über ein Symbol, welches über die Abkürzung 327 "am" und "pm" anzeigt, ob es sich um die Uhrzeit am Vormittag oder am Nachmittag handelt. So kann beispielsweise das Symbol "am" angekreuzt werden und zusätzlich ein Strich bei 3:00 gezogen werden, sodass klar ist, dass es sich bei der Uhrzeit um 3:00 Uhr nachmittags, also um 15:00 Uhr handelt. Zusätzlich wird über das Markieren von einem der sieben Ankreuzbereiche 124 klargestellt, an welchem Tag der Einwegfilter zu dieser entsprechenden Uhrzeit eingesetzt wurde.

Der Einwegfilter 300 mit seinem Aufdruck 320 ist daher besonders vorteilhaft für die Verwendung eines Einwegfilters, der nur wenige Stunden, insbesondere weniger als 48 Stunden, vorzugsweise weniger als 24 Stunden, genutzt werden sollte. Für derartige Filter ist eine Angabe, die eine zeitliche Auflösung von einzelnen Stunden erlaubt, und nicht nur von Tagen, als Kennzeichnungsmöglichkeit auf dem Aufdruck 320 vorgesehen.

Der Uhrenbereich 325 ist ein runder Uhrenbereich in dem dargestellten Ausführungsbeispiel. In einem alternativen Ausführungsbeispiel ist der Uhrenbereich ein rechteckiger Uhrenbereich und/oder ein Freitextfeld, über den die aktuelle Uhrzeit auf dem Aufdruck 320 aufgeschrieben werden kann. In diesem alternativen nicht dargestellten Ausführungsbeispiel ist zwar ein größerer manueller Aufwand durch das Eintragen der Uhrzeit notwendig, allerdings hat der Einwegfilter dabei dennoch den Vorteil, dass nicht das aktuelle Datum per Hand eingetragen werden muss. Dies führt zu einem geringeren manuellen Aufwand und zusätzlich zu einem geringeren Risiko von Fehlern beim Eintragen des Einsatzdatums.

Zusätzlich weist der Aufdruck 320 auf seiner strukturierten Fläche 322 ein ikonographisches Symbol 328, nämlich einen stilisierten Stift benachbart zu den Ankreuzbereichen 124 auf. Durch dieses ikonographisches Symbol 128 wird indiziert, dass die daran angeschlossene Fläche, also die strukturierte Fläche 322, für den Anwender des Einwegfilters 300 beschreibbar ist.

Der in Fig. 4 dargestellte Einwegfilter 400 unterscheidet sich von dem Einwegfilter 300 ausschließlich durch seinen Aufdruck 420.

So weist die strukturierte Fläche 422 neben den Ankreuzbereichen 124 und dem Uhrenbereich 425 mehrere Produktkennzeichnungen 440, 442, 444, 446 auf. Diese Produktkennzeichnungen sind teilweise aufgrund regionaler regulatorischer Anforderungen vorgesehen. So kann eine Produktkennzeichnung die Art der Entsorgung, relevante Inhaltsstoffe des Materials, eine vorgesehene einmalige Verwendung des Produkts, eine Produktbezeichnung, eine Herstellerbezeichnung oder dergleichen angeben. Die Produktkennzeichnung 442 zeigt beispielsweise an, dass es sich bei dem dargestellten Einwegfilter um einen mechanischen Filter, vorliegend in Abgrenzung zum HME-Filter, handelt. In dem dargestellten Ausführungsbeispiel hat der dargestellte Einwegfilter 400 beispielsweise die Produktbezeichnung "SafeStar 55". Die Ziffern "55" indizieren hierbei ein Totraumvolumen des Filters, welches 55 ml beträgt.

Die strukturierte Fläche 422 ist dabei derart geformt, dass die dargestellte zweite Filteröffnung 119 zumindest teilweise zirkular von der strukturierten Fläche 422 umgeben ist. Dabei ist die strukturierte Fläche 420 eine zusammenhängende Fläche. Anders als die Fläche 320 ist der Uhrenbereich 425 zusammenhängend mit den weiteren Bereichen der strukturierten Fläche 420 ausgebildet und nur teilweise rund ausgestaltet.

Durch die zirkular umgebende Struktur des Aufdrucks 420 kann dieser Aufdruck schnell optisch erfasst werden, sodass auch schnell entsprechend markierte Bereiche und/oder zu markierende Bereiche das Aufdrucks 420 durch einen Anwender des Einwegfilters 400 erkannt werden.

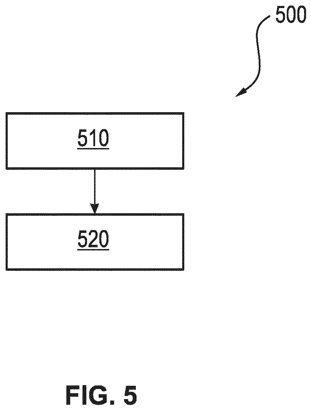

Fig. 5 zeigt ein Flussdiagramm eines Ausführungsbeispiels eines erfindungsgemäßen Verfahrens 500.

Das erfindungsgemäße Verfahren 500 ist zum Bedrucken eines Einwegfilters ausgebildet. Hierfür weist es die im Folgenden beschriebenen Schritte auf.

Ein erster Schritt 510 umfasst ein Bereitstellen eines Filtergehäuses.

Ein darauffolgender Schritt 520 umfasst ein Bedrucken des Filtergehäuses über ein Druckverfahren, insbesondere ein Tampondruckverfahren, derart, dass ein entsprechender Aufdruck eine strukturierte Fläche bildet, wobei die strukturierte Fläche beschreibbar ist, und wobei die strukturierte Fläche mindestens sieben Ankreuzbereiche umfasst.

Die beiden Schritte des Verfahrens 500 werden in der beschriebenen Reihenfolge ausgeführt. So wird erst das Filtergehäuse hergestellt und danach wird das Filtergehäuse erfindungsgemäß bedruckt über das Tampondruckverfahren. Der zeitliche Abstand zwischen den beiden Schritten ist abhängig von dem gewählten Herstellungsprozess. Spätestens bevor das Tampondruckverfahren ausgeführt wird, wird der entsprechenden Vorrichtung im Sinne des Schrittes 510 das Filtergehäuse bereitgestellt, um danach ein Aufdrucken der strukturierten Fläche durch die entsprechende Vorrichtung zu ermöglichen.

Das erfindungsgemäße Verfahren 500 zum Bedrucken des Einwegfilters kann ein Teilverfahren eines Herstellungsprozesses für den entsprechenden Einwegfilter sein. Im Rahmen dieses Herstellungsprozesses werden typischerweise weitere Schritte ausgeführt, wie beispielsweise das Einsetzen des Filtermaterials in den Einwegfilter und dergleichen. Besonders vorteilhaft wird das Bedrucken gemäß Schritt 520 als letzter Prozessschritt ausgeführt. Dadurch können Gehäuseeinzelteile für alle Filtervarianten als gleiche Teile ausgeführt werden, ehe die Verwendung über das Einsetzen des entsprechenden Filtermaterials festgelegt wird, so dass danach das erfindungsgemäße Bedrucken erfolgt. Derartige Verfahrensschritte können besonders vorteilhaft automatisiert durchgeführt werden.

### Bezugszeichenliste

- 100, 300, 400: Einwegfilter
- 110: Filtergehäuse
- 112: Filtermaterial
- 114: erste Gehäusekomponente
- 115: erster Anschluss
- 116: zweite Gehäusekomponente
- 117: zweiter Anschluss
- 118: erster Filteröffnung
- 119: zweite Filteröffnung
- 120, 320, 420: Aufdruck
- 122, 322, 422: strukturierte Fläche
- 124: Ankreuzbereich
- 130: Drainageverschluss
- 325,425: Uhrenbereich
- 326: Stundenangabe
- 327: Abkürzung
- 328: ikonographisches Symbol
- 440, 442, 444, 446: Produktkennzeichnung
- 500: Verfahren
- 510, 520: Verfahrensschritte

## Patentansprüche

1. Einwegfilter (100) zur Verwendung in einem Atemkreis eines Beatmungsgerätes, mit
- einem Filtergehäuse (110), welches ein Filtermaterial (112) des Einwegfilters (100) umgibt, wobei das Filtergehäuse (110) eine erste Filteröffnung (118) und eine zweite Filteröffnung (119) zum jeweiligen Anschluss eines gasführenden Bauteils, insbesondere eines jeweiligen Schlauchs, für ein durch den Einwegfilter (100) zu führendes Gas aufweist, wobei das Filtermaterial (112) zwischen der ersten Filteröffnung (118) und der zweiten Filteröffnung (119) angeordnet ist, **dadurch gekennzeichnet, dass** der Einwegfilter (100) einen Aufdruck (120) auf dem Filtergehäuse (110) aufweist, der über ein Druckverfahren, insbesondere ein Tampondruckverfahren, auf dem Filtergehäuse (110) angeordnet ist, und der eine strukturierte Fläche (122) bildet, wobei die strukturierte Fläche (122) beschreibbar ist, und wobei die strukturierte Fläche (122) mindestens sieben Ankreuzbereiche (124) umfasst.

2. Einwegfilter (100) gemäß Anspruch 1, wobei die strukturierte Fläche (122) eine derartige Rauigkeit aufweist, dass sie mit einem Kugelschreiber beschreibbar ist.

3. Einwegfilter (300) gemäß Anspruch 1 oder 2, wobei die strukturierte Fläche (322) zusätzlich zu den sieben Ankreuzbereichen (124) einen Uhrenbereich (325), insbesondere einen runden Uhrenbereich, umfasst, über den eine Uhrzeit auf der strukturierten Fläche (322) angestrichen werden kann.

4. Einwegfilter (400) gemäß mindestens einem der vorhergehenden Ansprüche, wobei die strukturierte Fläche (422) eine zusammenhängende Fläche ist.

5. Einwegfilter (400) gemäß Anspruch 4, wobei die strukturierte Fläche (422) eine die erste oder die zweite Filteröffnung (118, 119) zumindest teilweise zirkular umgebende Fläche ist.

6. Einwegfilter (400) gemäß mindestens einem der vorhergehenden Ansprüche, wobei die strukturierte Fläche (422) eine Produktkennzeichnung (440, 442, 444, 446) für den Einwegfilter (400) umfasst.

7. Einwegfilter (100) gemäß mindestens einem der vorhergehenden Ansprüche, wobei eine Farbe des Aufdrucks (120) einen Filter-Typ des Einwegfilters (100) aus einer vorbestimmten Gruppe von Filter-Typen indiziert.

8. Einwegfilter (300) gemäß mindestens einem der vorhergehenden Ansprüche, wobei die strukturierte Fläche (322) ein ikonographisches Symbol (328) umfasst, das eine Beschreibbarkeit dieser Fläche für einen Anwender des Einwegfilters (300) anzeigt.

9. Einwegfilter (100) gemäß mindestens einem der vorhergehenden Ansprüche, wobei der Einwegfilter (100) ein HME-Filter, ein elektrostatischer Filter und/oder ein mechanischer Filter ist.

10. Verfahren (500) zum Bedrucken eines Einwegfilters (100) nach einem der Ansprüche 1 bis 9, aufweisend die Schritte
- Bereitstellen des Filtergehäuses (110),
- Bedrucken des Filtergehäuses (110) über ein Druckverfahren, insbesondere ein Tampondruckverfahren, derart, dass ein entsprechender Aufdruck (120) eine strukturierte Fläche (122) bildet, wobei die strukturierte Fläche (122) beschreibbar ist, und wobei die strukturierte Fläche (122) mindestens sieben Ankreuzbereiche (124) umfasst.

## Claims

1. Disposable filter (100) for use in a breathing circuit of a ventilator, the disposable filter comprising:
- a filter housing (110), which surrounds a filter material (112) of the disposable filter (100), the filter housing (110) having a first filter opening (118) and a second filter opening (119) for the respective attachment of a gas-carrying component, in particular a respective hose, for a gas that is to be routed through the disposable filter (100), the filter material (112) being arranged between the first filter opening (118) and the second filter opening (119), **characterized in that** the disposable filter (100) has, on the filter housing (110), an imprint (120) which is applied to the filter housing (110) by means of a printing process, in particular a pad printing process, and which forms a structured surface (122), the structured surface (122) being able to be written on, and the structured surface (122) comprising at least seven marking regions (124).

2. Disposable filter (100) according to Claim 1, wherein the structured surface (122) has a roughness such that it is able to be written on with a pen.

3. Disposable filter (300) according to Claim 1 or 2, wherein the structured surface (322) comprises, in addition to the seven marking regions (124), a clock region (325), in particular a round clock region, by means of which a time can be marked on the structured surface (322).

4. Disposable filter (400) according to at least one of the preceding claims, wherein the structured surface (422) is a contiguous surface.

5. Disposable filter (400) according to Claim 4, wherein the structured surface (422) is a surface at least partially enclosing the first or the second filter opening (118, 119) in a circle.

6. Disposable filter (400) according to at least one of the preceding claims, wherein the structured surface (422) comprises a product identification (440, 442, 444, 446) for the disposable filter (400).

7. Disposable filter (100) according to at least one of the preceding claims, wherein a colour of the imprint (120) indicates a filter type of the disposable filter (100) from a predetermined group of filter types.

8. Disposable filter (300) according to at least one of the preceding claims, wherein the structured surface (322) comprises an iconographic symbol (328), which indicates to a user of the disposable filter (300) that this surface is able to be written on.

9. Disposable filter (100) according to at least one of the preceding claims, wherein the disposable filter (100) is an HME filter, an electrostatic filter and/or a mechanical filter.

10. Method (500) for printing on a disposable filter (100) according to one of Claims 1 to 9, said method comprising the steps of:
- providing the filter housing (110),
- printing on the filter housing (110) by means of a printing process, in particular a pad printing process, in such a way that a corresponding imprint (120) forms a structured surface (122), wherein the structured surface (122) is able to be written on, and wherein the structured surface (122) comprises at least seven marking regions (124).

## Revendications

1. Filtre jetable (100) conçu pour être utilisé dans un circuit respiratoire d'un respirateur, comprenant
- un boîtier (110) qui entoure un matériau filtrant (112) dudit filtre jetable (100), ledit boîtier (110) du filtre comportant un premier orifice de filtration (118) et un second orifice de filtration (119) affectés au raccordement respectif d'une pièce structurelle de guidage de gaz, en particulier d'un tuyau souple respectif destiné à un gaz devant être guidé à travers ledit filtre jetable (100), le matériau filtrant (112) étant interposé entre le premier orifice de filtration (118) et le second orifice de filtration (119),
**caractérisé par le fait que** le filtre jetable (100) est muni, sur le boîtier (110) dudit filtre, d'une mention imprimée (120) qui est appliquée sur ledit boîtier (110) du filtre à l'aide d'un procédé d'impression, notamment d'un procédé de tampographie, et qui forme une surface structurée (122), laquelle surface structurée (122) peut être revêtue d'inscriptions, et ladite surface structurée (122) incluant au moins sept zones cochables (124).

2. Filtre jetable (100) selon la revendication 1, la surface structurée (122) étant douée d'une rugosité telle qu'elle puisse être revêtue d'inscriptions à l'aide d'un stylo à bille.

3. Filtre jetable (300) selon la revendication 1 ou 2, la surface structurée (322) étant pourvue, en plus des sept zones cochables (124), d'un cadran horaire (325) et notamment d'un cadran horaire circulaire par l'intermédiaire duquel une heure d'horloge peut être marquée d'un repère sur ladite surface structurée (322).

4. Filtre jetable (400) selon au moins l'une des revendications précédentes, la surface structurée (422) étant une surface cohérente.

5. Filtre jetable (400) selon la revendication 4, la surface structurée (422) étant une surface qui entoure circulairement, au moins en partie, le premier ou le second orifice de filtration (118, 119).

6. Filtre jetable (400) selon au moins l'une des revendications précédentes, la surface structurée (422) incluant un identifiant de produit (440, 442, 444, 446) dédié audit filtre jetable (400).

7. Filtre jetable (100) selon au moins l'une des revendications précédentes, une couleur de la mention imprimée (120) étant indicative d'un type dudit filtre jetable (100) au sein d'un groupe prédéterminé de types de filtres.

8. Filtre jetable (300) selon au moins l'une des revendications précédentes, la surface structurée (322) incluant un symbole iconographique (328) qui indique, à un utilisateur dudit filtre jetable (300), une aptitude de cette surface à recevoir des inscriptions.

9. Filtre jetable (100) selon au moins l'une des revendications précédentes, ledit filtre jetable (100) étant un filtre HME, un filtre électrostatique et/ou un filtre mécanique.

10. Procédé (500) dévolu à l'impression d'un filtre jetable (100) conforme à l'une des revendications 1 à 9, comprenant les étapes consistant à
- apprêter le boîtier (110) dudit filtre,
- porter des inscriptions sur ledit boîtier (110) du filtre à l'aide d'un procédé d'impression, notamment d'un procédé de tampographie, de façon telle qu'une mention imprimée (120) correspondante forme une surface structurée (122), laquelle surface structurée (122) peut être revêtue d'inscriptions, et ladite surface structurée (122) incluant au moins sept zones cochables (124).
